# EUROPEAN PATENT APPLICATION

(11) **EP 2 510 880 A1**
(43) Date of publication of application: **17.10.2012**
(21) Application number: 12163975.1
(22) Date of filing: 12.04.2012
(51) Int. Cl.: A61B 8/00

(54) **Ultrasound diagnostic system**

(30) Priority: 12.04.2011 KR 20110033955; 22.09.2011 KR 20110095916
(71) Applicant: Samsung Medison Co., Ltd., Kangwon-do 250-875 (KR)
(72) Inventor: Lee, Yun Ju, Seoul (KR); Nam, Sang Gyu, Seoul (KR)
(74) Representative: Schmid, Wolfgang

(57) **Abstract**

An ultrasound diagnostic system includes a portable ultrasound diagnostic device and an extended docking device to which the portable ultrasound diagnostic device is detachably mounted, wherein at least one of probes and channels are extended when the portable ultrasound diagnostic device is mounted to the extended docking device. Also, the ultrasound diagnostic system may further include an indoor ultrasound diagnostic device including a portable docking part, and the portable ultrasound diagnostic device may include a cart-based docking part and be connected to the indoor ultrasound diagnostic device. The ultrasound diagnostic system may enhance portability of the portable ultrasound diagnostic device and simultaneously achieves superior ultrasound diagnostic performance and quality also in the portable ultrasound diagnostic device through extension of the probes, signal channels, diagnostic items, or diagnostic performance, as occasion demands.

## Description

### CROSS-REFERENCE TO RELATED APPLICATION

This application claims the benefit of Korean Patent Applications No. P2011-0033955 filed on April 12, 2011, and No. P2011-0095916 filed on September 22, 2011 in the Korean Intellectual Property Office, the disclosures of which are incorporated herein by reference.

### BACKGROUND

### 1. Field

The present subject matter relates to an ultrasound diagnostic system capable of enhancing portability of a portable ultrasound diagnostic device and simultaneously superior ultrasonic diagnostic performance and quality of the portable ultrasound diagnostic device through extension of probes and/or signal channels as occasion demands.

### 2. Description of the Related Art

Ultrasound diagnostic systems have noninvasive and nondestructive characteristics. Ultrasound diagnostic systems provide medical doctors with high resolution images of inner tissues of objects, without a need for surgical operations to directly incise the objects to observe the objects. The noninvasiveness and high resolution images of the ultrasound diagnostic systems promote wide use of the ultrasound diagnostic systems in the field of medicine to obtain information on objects within a human body.

Such ultrasound diagnostic systems, however, are very large and heavy, so need to be fixed to particular places. Since even small size ultrasound diagnostic systems are 10 kg or more in weight, they are not easy to carry. Meanwhile, since ultrasound diagnostic systems are used in emergency rooms, operation rooms, or any other places, where the ultrasound diagnostic systems must frequently be moved, the ultrasound diagnostic systems need to be small. Needs for small size ultrasound diagnostic devices encourage development of portable ultrasound diagnostic devices.

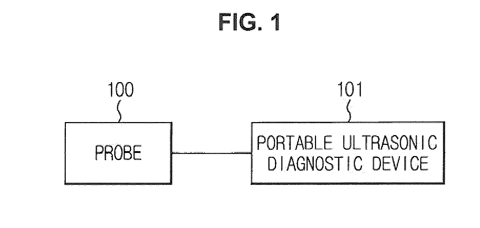

FIG. 1 is a view schematically illustrating a portable ultrasound diagnostic device according to the related art. As shown in FIG. 1, one probe 101 is general mounted at the conventional portable ultrasound diagnostic device 100 for portability, and the portable ultrasound diagnostic device 100 has a limited number of signal channels corresponding to the probe 101. Although the conventional portable ultrasound diagnostic device 100 is suitable to promote convenience of portability or movement, there is an extreme limit as to the number of probes to be mounted at the same. Consequently, since only a particular sort of probe may be mounted at the conventional portable ultrasound diagnostic device 100 due to limitations on number of probes mounted on the ultrasound diagnostic device 100, the conventional ultrasound diagnostic device is used for limited purposes of particular diagnosis.

In addition, the conventional portable ultrasound diagnostic device has a limit as to the number of signal channels to be provided for the sake of portability, which thereby deteriorates resolution and quality of produced ultrasound images.

Furthermore, since portable ultrasound diagnostic devices have many limitations in terms of size, weight, power consumption, and the like, the portable ultrasound diagnostic devices show poor ultrasound diagnostic performance and quality compared with cart-based ultrasound diagnostic devices.

### SUMMARY

An aspect of the present invention encompasses an ultrasound diagnostic system capable of enhancing portability of a portable ultrasound diagnostic device and achieving superior ultrasound diagnostic performance and quality through extension of probes and/or signal channels as occasion demands.

Another aspect of the present invention relates to an ultrasound diagnostic system. The ultrasound diagnostic system includes a portable ultrasound diagnostic device and an extended docking device to which the portable ultrasound diagnostic device is detachably mounted, wherein the portable ultrasound diagnostic device attains extension of at least one probes and extension of channels when being mounted at the extended docking device.

The extended docking device may include a docking interface for connection with the portable ultrasound diagnostic device, at least one probe port including probe ports to respectively receive probes, and a channel board capable of extending signal channels corresponding to the probe ports.

The portable ultrasound diagnostic device may attain extension of at least one probes and channels through the at least one probe port and/or the channel board when being mounted at the extended docking device.

The portable ultrasound diagnostic device may include a secondary battery as a power supply unit, and the extended docking device may include a charging circuit part to charge the secondary battery during mounting of the portable ultrasound diagnostic device.

The portable ultrasound diagnostic device may include an extended docking part for connection with the extended docking device, and the extended docking part may include a power docking connector and a control docking connector.

The ultrasound diagnostic system may further include an indoor ultrasound diagnostic device comprising a portable docking part for connection with the portable ultrasound diagnostic device.

The portable ultrasound diagnostic device may include a cart-based docking part for connection with the indoor ultrasound diagnostic devices, and be connected to the indoor ultrasound diagnostic device through the cart-based docking part and the portable docking part.

The cart-based docking part may include a power docking connector and a control docking connector.

The indoor ultrasound diagnostic device may include an electrocardiogram (ECG) port so as to analyze an electrocardiogram signal.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a view schematically illustrating a portable ultrasound diagnostic device according to the related art;
FIG. 2 is a control block diagram illustrating an ultrasound diagnostic system according to a first embodiment of the present invention;
FIG. 3 is a side view illustrating the ultrasound diagnostic system of the first embodiment;
FIG. 4 is a control block diagram illustrating an extended docking part of a portable ultrasound diagnostic device in the ultrasound diagnostic system of the first embodiment;
FIG. 5 is a perspective view illustrating an ultrasound diagnostic system according to a second embodiment of the present invention; and
FIG. 6 is a control block diagram illustrating the ultrasound diagnostic system according to the second embodiment.

### DETAILED DESCRIPTION

Reference will now be made in detail to the embodiments of the present invention, examples of which are illustrated in the accompanying drawings, wherein like reference numerals refer to like element throughout.

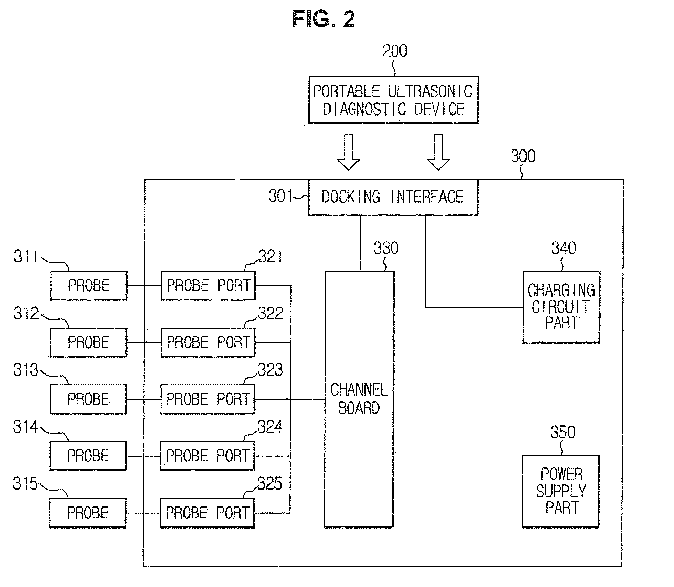

FIG. 2 is a control block diagram illustrating an ultrasound diagnostic system according to the first embodiment.

As shown in FIG. 2, the ultrasound diagnostic system according to the first embodiment includes a portable ultrasound diagnostic device 200 and an extended docking device 300 to detachably mount the portable Ultrasound diagnostic device 200. The extended docking device 300 may include a docking interface 301 for connection with the portable Ultrasound diagnostic device 200, at least one probe port including probe ports 321 to 325 to respectively receive probes 311 to 315, a channel board 330 capable of extending signal channels corresponding to the probe ports 321 to 325, and a charging circuit part 340 to charge a secondary battery (not shown) during mounting of the portable ultrasound diagnostic device 200.

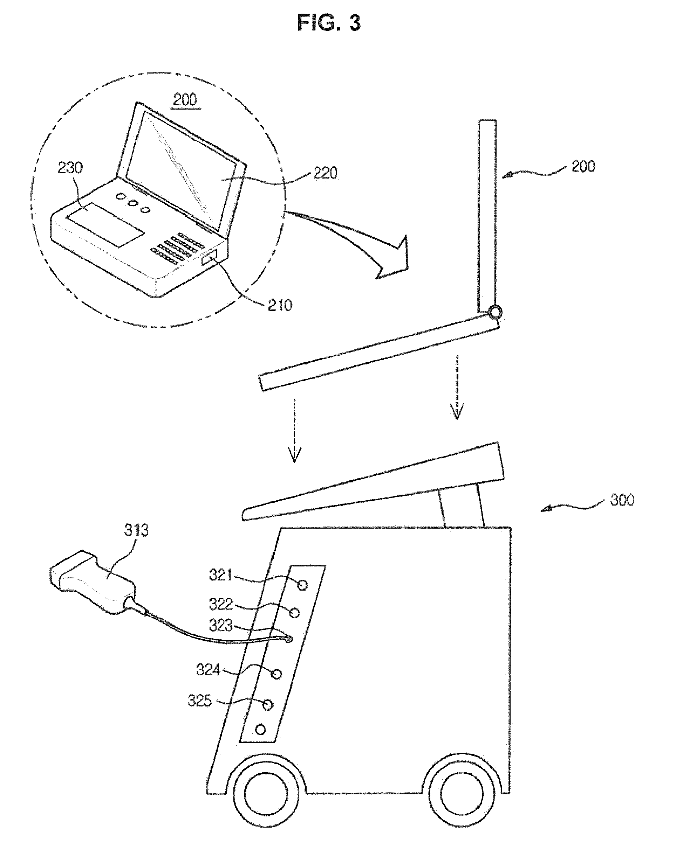

FIG. 3 is a side view illustrating the portable ultrasound diagnostic device 200 and the extended docking device 300 mounting the same according to the first embodiment.

Referring to FIG. 3, in the case of requiring portability of the ultrasound diagnostic device for outdoor diagnosis, ultrasound diagnosis may be easily executed using only the portable ultrasound diagnostic device 200 including a display part 220, an input part 230, and a probe port 210. On the other hand, in the case of requiring high quality images or additional probe ports besides the probe port 210 included in the portable ultrasound diagnostic device 200, the portable ultrasound diagnostic device 200 may be mounted at the extended docking device 300.

Hereinafter, operation of the ultrasound diagnostic system according to the exemplary embodiment of the present invention will be described in detail with reference to FIGS. 2 and 3.

The ultrasound diagnostic system of the exemplary embodiment has a structure in which the portable ultrasound diagnostic device 200 may be coupled to the extended docking device 300 capable of extending the probes and/or signal channels. That is, the ultrasound diagnostic system of the first embodiment is provided with the extended docking device 300 at an lower part of the system, to which the portable ultrasound diagnostic device 200 is coupled or docked, as shown in FIGS. 2 and 3. In accordance with such a structure, the extension of the probes and/or signal channels may be achieved during docking of the two devices, and the specific description thereof is as follows.

First, the portable ultrasound diagnostic device 200 may be detachably mounted at the extended docking device 300 through the docking interface 301, as shown in FIG. 2. In this case, an array pin type connector (not shown) may be used for coupling or mounting the portable ultrasound diagnostic device 200 to the extended docking device 300. Since the portable ultrasound diagnostic device 200 may be easily attached to or detached from the extended docking device 300, ultrasound diagnosis may be easily performed by detaching the portable ultrasound diagnostic device 200 from the extended docking device 300, in case of requiring simple portability while not requiring ultrasound images of high quality in the ultrasound diagnostic device.

Alternatively, in case of requiring a certain level of portability while requiring ultrasound images of high quality or performance in the ultrasound diagnostic device, ultrasound diagnosis may be achieved by mounting the portable ultrasound diagnostic device 200 to the extended docking device 300. That is, the extended docking device 300 includes a plurality of probe ports 321 to 325 which receive a plurality of probes 311 to 315 respectively, as shown in FIGS. 2 and 3. Here, various types of probes may be applied as the plural probes 311 to 315 depending on diagnostic purposes. For example, the various types of probes, such as a linear probe for carotid diagnosis, a convex probe for abdominal diagnosis, a phased array probe for heart diagnosis, and the like, may be applied. Accordingly, a range of a usable probe may extend through the plural probe ports 321 to 325 included in the extended docking device 300. The number of the probe ports are not limited to five, and any number of probe ports may be provided.

The extended docking device 300 includes the channel board 330 having the signal channels corresponding to the probe ports 321 to 325, and usable channels may increase in the portable ultrasound diagnostic device 200 through the signal channels included in the channel board 330. The number of signal channels included in the channel board 330 may increase according to desired resolution and quality of the ultrasound images.

As such, when the portable ultrasound diagnostic device 200 is mounted to the extended docking device 300, at least one of probes and channels may be extended through the plural probe ports 321 to 325 and/or the channel board 330.

In order to achieve the extension of the probes and/or the extension of the signal channels, the portable ultrasound diagnostic device 200 should receive software of the extended docking device 300, for maintaining compatibility between the portable ultrasound diagnostic device 200 and the extended docking device 300.

Meanwhile, the portable ultrasound diagnostic device 200 may include the secondary battery (not shown) as a power supply unit, and the extended docking device 300 may include the charging circuit part 340 to charge the secondary battery during mounting of the portable ultrasound diagnostic device 200. That is, the portable ultrasound diagnostic device 200 has the secondary battery (not shown) used as the power supply unit for portability. When the portable ultrasound diagnostic device 200 is mounted to the extended docking device 300, the secondary battery is automatically charged through the charging circuit part 340.

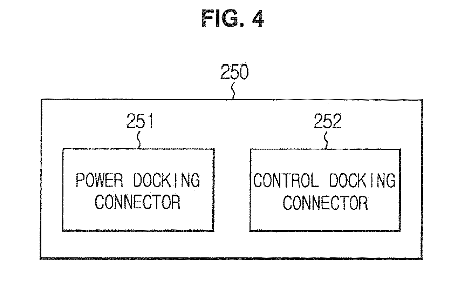

Although not specifically described in the illustrated embodiment, the extended docking device 300 may include a hardware element for the channel extension, a circuit for generation and control of a variety of pulses, a power supply hardware element including a power supply part 350, a heat generating unit, etc. **FIG. 4** is a control block diagram illustrating docking connectors of the portable ultrasound diagnostic device in the ultrasound diagnostic system according to the exemplary embodiment.

In the ultrasound diagnostic system according to the exemplary embodiment, the array pin type connector (not shown) may be used for coupling or mounting the portable ultrasound diagnostic device 200 as the upper device to the extended docking device 300 as the lower device, as described above. As shown in FIG. 4, the portable ultrasound diagnostic device 200 may include an extended docking part 250 to dock the extended docking device 300, and the extended docking part 250 may include a power docking connector 251 to couple a power between the portable Ultrasound diagnostic device 200 and the extended docking device 300, and a control docking connector 252 to control operations between the portable ultrasound diagnostic device 200 and the extended docking device 300.

When the power docking connector 251 is separated from the control docking connector 252 as described above, power docking and control docking may be separately performed, thereby removing noise generated when one connector is used. As a result, it is possible to prevent performance deterioration of the ultrasound diagnostic device.

There is a case where a portable ultrasound diagnostic device is used independently. Alternatively, there is another case where a portable ultrasound diagnostic device is used with being mounted to the extended docking device 300 as described above, to perform outdoor diagnosis. Then, a diagnostic result obtained from the portable ultrasound diagnostic device is analyzed as a more vivid image. An additional function, which is absent from the portable ultrasound diagnostic device, is further performed on the diagnostic result inside a room.

Accordingly, a portable ultrasound diagnostic device 400 of an ultrasound diagnostic system according to another exemplary embodiment may further include a cart-based docking part 480 connected to an indoor ultrasound diagnostic device 500. The ultrasound diagnostic system may further include the indoor ultrasound diagnostic device 500 having a portable docking part 580 connected to the portable ultrasound diagnostic device 400.

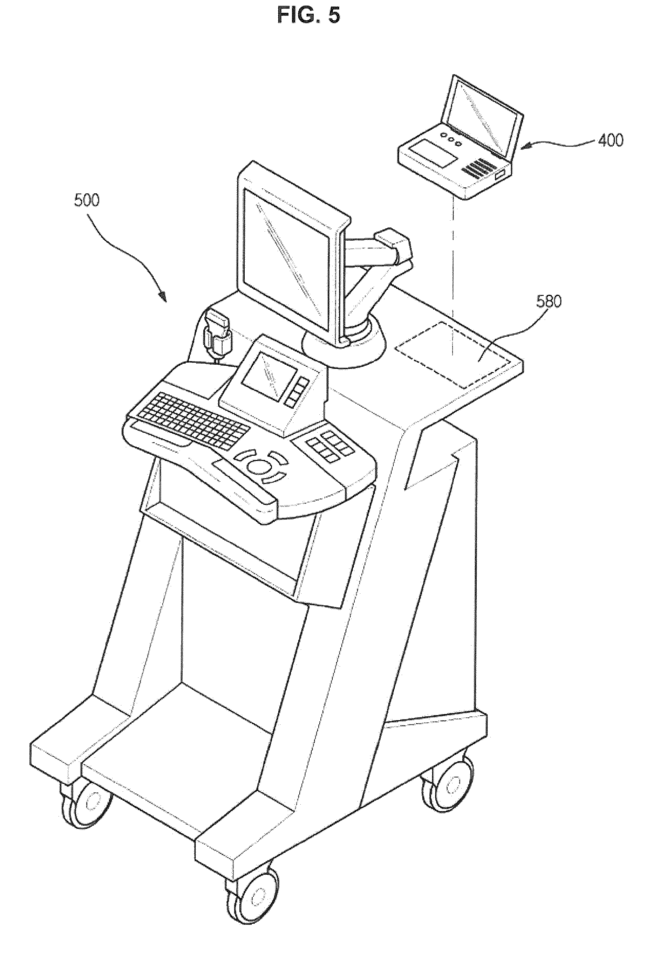

FIG. 5 is a perspective view illustrating an entire configuration of the ultrasound diagnostic system according to the second embodiment.

As shown in FIG. 5, the ultrasound diagnostic device used indoors or the indoor Ultrasound diagnostic device 500 is an ultrasound diagnostic device, which is generally used for ultrasound diagnosis and is not portable. Such an indoor ultrasound diagnostic device 500 is often referred to as a cart-based device. The Ultrasound diagnostic device 500 need not necessarily be used indoors, but is referred to as an indoor Ultrasound diagnostic device for convenience. Since all components in the illustrated embodiment are similar to components of a general ultrasound diagnostic device, except for the portable docking part 580 of the indoor ultrasound diagnostic device 500 utilized in tha exemplary embodiments, no description thereof will be given in detail. The indoor ultrasound diagnostic device 500 has little limit in terms of size, weight, power consumption, and the like, compared with the portable ultrasound diagnostic device 400. Thus, the indoor ultrasound diagnostic device 500 may be developed as a high performance device having various diagnostic items. When the portable ultrasound diagnostic device 400 is mounted to the indoor ultrasound diagnostic device 500, the portable ultrasound diagnostic device 400 may produce a high performance. Here, a position at which the portable ultrasound diagnostic device 400 is mounted to the indoor ultrasound diagnostic device 500 is not limited to that shown in FIG. 5. The portable ultrasound diagnostic device 400 may be located at any position of the indoor ultrasound diagnostic device as a user may simultaneously and easily utilize the portable ultrasound diagnostic device 400 and the indoor ultrasound diagnostic device 500.

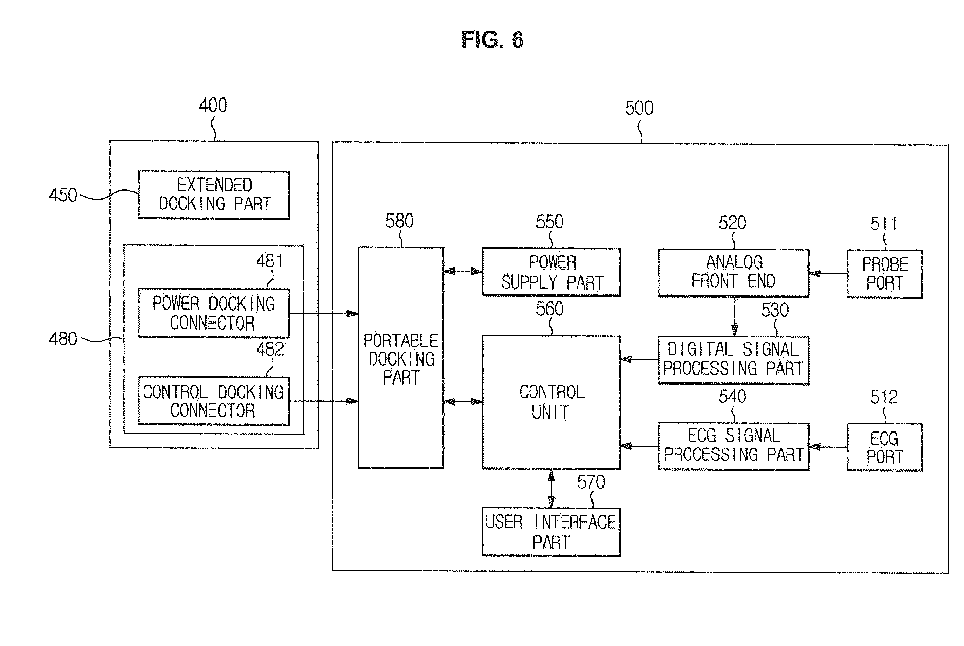

FIG. 6 is a control block diagram illustrating the ultrasound diagnostic system according to the second embodiment.

Referring to FIG. 6, the ultrasound diagnostic system according to the second embodiment includes the portable ultrasound diagnostic device 400 having the cart-based docking part 480 and the indoor ultrasound diagnostic device 500 having the portable docking part 580.

The portable ultrasound diagnostic device 400 may include an extended docking part 450 so as to be connected with the above-mentioned extended docking device 300. A power docking connector 481 may be separated from a control docking connector 482 as described above, thereby preventing performance deterioration due to noise generation. The portable ultrasound diagnostic device 400 shown in the control block diagram of FIG. 6 illustrates only a portion relative to docking, and no description will be given in conjunction with general functions of the portable ultrasound diagnostic device 400.

The indoor ultrasound diagnostic device 500 includes a probe port 511 connected to an ultrasound probe, an analog front end (AFE) 520 to convert analog signals transmitted from the probe port 511 into digital signals, a digital signal processing part 530 to process the converted digital signals, and a control unit 560 to perform various types of controls within the ultrasound diagnostic device 500 using the processed digital signals. The indoor ultrasound diagnostic device 500 further includes a portable docking part 580 connected to the portable ultrasound diagnostic device 400, a user interface part 570, and a power supply part 550 connected to a power supply unit of the portable ultrasound diagnostic device 400 for charging the power supply unit.

The indoor ultrasound diagnostic device 500 may also include an electrocardiogram (ECG) port 512 so as to measure an electrocardiogram in addition to diagnosis using the probe. The indoor ultrasound diagnostic device 500 enables setting of various modes such as a continuous wave (CW) Doppler mode, a color Doppler mode, or a power Doppler mode so as to analyze an ultrasound image photographed by the portable ultrasound diagnostic device 400 in a desired mode.

The portable ultrasound diagnostic device 400 may be connected or coupled to the indoor ultrasound diagnostic device 500, thereby enabling utilization of various functions of the indoor ultrasound diagnostic device 500. As described above, the ultrasound diagnostic system according to the second embodiment may enhance portability of the portable ultrasound diagnostic device and simultaneously achieves superior ultrasound diagnostic performance and quality also in the portable ultrasound diagnostic device through the extension of the probes, signal channels, diagnostic items, or diagnostic performance, as occasion demands.

As is apparent from the above description, the present invention provides an ultrasound diagnostic system capable of enhancing portability of a portable ultrasound diagnostic device and simultaneously achieves superior ultrasound diagnostic performance and quality also in the portable ultrasound diagnostic device through extension of probes and/or signal channels as occasion demands.

Also, a portable ultrasound diagnostic device may be connected to a cart-based body through a docking device in case of not requiring portability, thereby serving as a high performance ultrasound diagnostic device. On the other hand, the portable ultrasound diagnostic device may be separated from the docking device in case of requiring portability, thereby serving as a portable device.

Furthermore, in the case of connecting a portable ultrasound diagnostic device to a docking device or an indoor ultrasound diagnostic device, a power docking connector may be separated from a control docking connector, thereby preventing performance deterioration due to noise generation.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. An ultrasound diagnostic system comprising:
a portable Ultrasound diagnostic device; and
an extended docking device to which the portable Ultrasound diagnostic device is detachably mounted,
wherein at least one of probes and channels are extended with mounting of the portable Ultrasound diagnostic device to the extended docking device.

2. The Ultrasound diagnostic system according to claim 1, wherein the extended docking device comprises:
a docking interface for connection with the portable ultrasound diagnostic device;
at least one probe port comprising probe ports to respectively receive probes; and
a channel board capable of extending signal channels corresponding to the probe ports.

3. The ultrasound diagnostic system according to claim 2, wherein the portable Ultrasound diagnostic device attains at least one of the probes and channels are extended through the at least one probe port and/or the channel board with being mounted to the extended docking device.

4. The ultrasound diagnostic system according to claim 1, wherein the portable ultrasound diagnostic device comprises a secondary battery as a power supply unit, and the extended docking device comprises a charging circuit part to charge the secondary battery during mounting of the portable ultrasound diagnostic device.

5. The ultrasound diagnostic system according to claims 1, wherein:
the portable ultrasound diagnostic device comprises an extended docking part for connection with the extended docking device;
the extended docking part comprises a power docking connector to couple power between the portable ultrasound diagnostic device and the extended docking device, and a control docking connector to control operations between the portable ultrasound diagnostic device and the extended docking device.

6. The ultrasound diagnostic system according to claim 1, further comprising an indoor ultrasound diagnostic device comprising a portable docking part for connection with the portable ultrasound diagnostic device.

7. The ultrasound diagnostic system according to claim 6, wherein the portable ultrasound diagnostic device comprises a cart-based docking part for connection with the indoor ultrasound diagnostic device, and is connected to the indoor ultrasound diagnostic device through the cart-based docking part and the portable docking part.

8. The ultrasound diagnostic system according to claim 7, wherein the cart-based docking part comprises a power docking connector to couple power between the portable ultrasound diagnostic device and the indoor ultrasound diagnostic device, and a control docking connector to control operations the portable ultrasound diagnostic device and the indoor ultrasound diagnostic device.

9. The ultrasound diagnostic system according to claim 7, wherein the indoor ultrasound diagnostic device comprises an electrocardiogram (eco) port to analyze an electrocardiogram signal.
